# EUROPÄISCHE PATENTSCHRIFT

(11) **EP 1 999 102 B1**
(45) Veröffentlichungstag und Bekanntmachung des Hinweises auf die Patenterteilung: **14.12.2011**
(21) Anmeldenummer: 07727106.2
(22) Anmeldetag: 20.03.2007
(51) Int. Cl.: C07C 213/04, C07C 217/08, B01D 3/00

(54) **VERFAHREN ZUR HERSTELLUNG VON N,N-DIMETHYLAMINOETHOXYETHANOL**
PROCESS FOR PREPARING N,N-DIMETHYLAMINOETHOXYETHANOL
PROCEDE DE FABRICATION DE N,N-DIMETHYLAMINOETHOXYETHANOL

(30) Priorität: 20.03.2006 EP 06111382; 06.07.2006 EP 06116712
(43) Veröffentlichungstag der Anmeldung: 10.12.2008
(73) Patentinhaber: BASF SE, 67056 Ludwigshafen (DE)
(72) Erfinder: HAESE, Frank, 63128 Dietzenbach (DE); MELDER, Johann-Peter, 67459 Böhl-Iggelheim (DE); KRAUSE, Alfred, 67346 Speyer (DE); PAPE, Frank-Friedrich, 67259 Kleinniedesheim (DE); STEIN, Bernd, 64665 Alsbach-Hähnlein (DE)
(86) Internationale Anmeldenummer: PCT/EP2007/052629
(87) Internationale Veröffentlichungsnummer: WO 2007/107557

(56) Entgegenhaltungen:
- EP-A- 1 203 780
- DE-A1- 4 414 879

## Beschreibung

Die Erfindung betrifft ein Verfahren zur Herstellung von *N*,*N*-Dimethylaminoethoxyethanol, eine Vorrichtung zu dessen Durchführung und die Verwendung von Nebenprodukten der Herstellung von *N*,*N* Dimethylaminoethanol in einem solchen Verfahren.

2-[2-(Dimethylamino)ethoxy]ethanol (*N*,*N*-Dimethylaminoethoxyethanol, DMAEE) ist eine kommerziell erhältliche Verbindung (BASF Corporation, USA), die beispielsweise als Zwischenprodukt bei der Synthese von Pharmawirkstoffen oder als Katalysator in der Polyurethanherstellung Anwendung findet.

Die Herstellung von DMAEE erfolgt zum einen durch Umsetzung von Diethylenglykol mit Dimethylamin an einem Katalysator (siehe z. B. EP-A 0 300 323, JP-A 62/051646, JP-A 08/143520 und JP-A 09/020735), zum anderen durch Umsetzung von *N*,*N* Dimethylethanolamin mit Ethylenoxid (siehe z. B. J.G. Cannon et al., Journal of Pharmaceutical Sciences 62 (1973) 830, US 3,853, 818 und EP-A 1 203 780).

Obwohl nach den genannten Verfahren teilweise großtechnisch gearbeitet wird, bleibt doch ein breiter Raum für Verbesserungen, beispielsweise bezüglich Ausbeuten, Selektivität, Reaktionszeiten oder einfacher Aufarbeitbarkeit.

Aufgabe der Erfindung ist es, ein Verfahren zur Herstellung von DMAEE bereitzustellen, das zumindest in Teilaspekten Vorteile gegenüber den bekannten Verfahren aufweist.

Es wurde nun gefunden, dass sich DMAEE bei der industriellen Herstellung von *N*,*N* Dimethylethanolamin in technisch verwertbaren Mengen bildet und in einfacher Weise bei der Reinigung des *N*,*N*- Dimethylethanolamins abgetrennt werden kann.

Gegenstand der Erfindung ist daher ein Verfahren zur Herstellung von *N*,*N*- Dimethylaminoethoxyethanol (DMAEE), wobei man
a) Dimethylamin und Ethylenoxid zur Reaktion bringt,
b) das entstandene Produktgemisch von *N*,*N*-Dimethylethanolamin und DMAEE destillativ auftrennt, wobei eine DMAEE-haltige Fraktion als Sumpfstrom erhalten wird, und
c) DMAEE aus der in (b) erhaltenen Fraktion destillativ abtrennt.

Weiterhin Gegenstand der Erfindung ist die Verwendung des Sumpfstroms einer Destillation der Reaktionsmischung von Dimethylamin und Ethylenoxid zur Gewinnung von DMAEE.

Ebenso Gegenstand der Erfindung ist eine Vorrichtung zur Durchführung des oben beschriebenen Verfahrens, enthaltend (I) einen Reaktor, vorzugsweise einen Rohrreaktor, mit Zuleitungen für Dimethylamin und Ethylenoxid und einer Ableitung für das Reaktionsprodukt, (II) eine Destillationsvorrichtung zur Anreicherung des DMAEE durch destillative Abtrennung von *N*,*N*-Dimethylethanolamin aus dem Reaktionsgemisch mit einer Ableitung für das DMAEE-haltige Sumpfprodukt am Fuß der Destillationsvorrichtung und (III) eine zur fraktionierten Destillation geeignete Vorrichtung, mit einer Zuleitung für das DMAEE-haltige Reaktionsgemisch am Fuß oder an der Seite und einer Ableitung für das destillierte DMAEE im Kopfbereich der Vorrichtung.

Mit dem erfindungsgemäßen Verfahren kann DMAEE in einfacher Weise und ohne aufwendige Synthese mit Katalysatoren und komplexen Produktgemischen erhalten werden. Vorteilhaft ist es auch, dass zum Erhalt einer farbstabilen Reinware eine Hydrierung an einem Edelmetallkontakt nicht notwendig ist.

Die Reaktion von Ethylenoxid und Dimethylamin zu hauptsächlich *N*,*N-*Dimethylethanolamin ist mit ihren Verfahrensparametern bekannt und wird großtechnisch zur Herstellung von *N*,*N*-Dimethylethanolamin verwendet. Einzelheiten dieses Reaktionsschrittes sind beispielsweise in der DE-A 44 14 879 beschrieben.

Das gebildete Reaktionsgemisch enthält im Allgemeinen 1 bis 6 Gew.-%, bevorzugt 2 bis 3 Gew.-%, DMAEE.

Die destillative Abtrennung des gebildeten DMAEE von der Hauptkomponente, *N*,*N-*Dimethylethanolamin, erfolgt vorteilhaft mit einer kontinuierlich oder diskontinuierlich betriebenen Kolonne in einem Temperaturbereich (Sumpf) von 40°C bis 150°C, bevorzugt von 40°C bis 90°C und bei einem Druck von 5 bis 1050 mbar, bevorzugt 5 bis 300 mbar, besonders bevorzugt 10 bis 150 mbar.

DMAEE ist im Sumpfstrom dieser Kolonne angereichert.

Zur Gewinnung von reinem DMAEE wird daher der gesammelte Sumpfstrom in einem folgenden Schritt fraktioniert destilliert.

Für diese Destillation eignen sich alle bekannten Kolonnentypen, wie z.B. Füllkörperkolonnen, Bodenkolonnen, Packungskolonnen und Trennwandkolonnen. Bevorzugt sind Packungskolonnen und Trennwandkolonnen. In einer weiteren bevorzugten Ausführung des Verfahrens erfolgt die Destillation von DMAEE bei niedrigeren Drücken, beispielsweise in einem Dünnschichtverdampfer, Fallfilmverdampfer oder Kurzwegverdampfer oder einem der letztgenannten Apparate mit aufgesetzter Kolonne beliebiger Art, z. B. einer Packungskolonne.

Die fraktionierte Destillation in einer Kolonne erfolgt im Allgemeinen in einem Temperaturbereich (Sumpf) von 40 bis 250°C, vorzugsweise von 135 bis 235°C, insbesondere bei 170 bis 200°C. Die Destillation wird im Allgemeinen unter vermindertem Druck, vorzugsweise in einem Bereich von 1 bis 1000 mbar, bevorzugt von 100 bis 500 mbar, besonders bevorzugt 400 mbar, durchgeführt. Das Rücklaufverhältnis beträgt im allgemeinen ca. 1,5 : 1 (Rücklauf zu Abnahme) bis 5 : 1.

In der weiteren bevorzugten Ausführung des Verfahrens in einem Dünnschichtverdampfer, Fallfilmverdampfer oder Kurzwegverdampfer erfolgt die Destillation bei Übergangstemperaturen von 40 bis 150°C, bevorzugt 40 bis 100°C, und unter einem Druck von 0,001 bis 1 mbar, bevorzugt 0,01 bis 0,1 mbar.

Dass bei sehr gutem Vakuum niedrigere Destillationstemperaturen möglich sind und dadurch das Produkt schonend destilliert werden kann, ist dem Fachmann bekannt. Überraschend wurde bei dieser Destillation gefunden, dass das Produkt DMAEE sogar bei relativ scharfen Destillationsbedingungen stabil bleibt (siehe Tabelle 1) und mit guten Ausbeuten und Reinheiten isoliert werden konnte.

Das in diesem Schritt erhaltene DMAEE hat im Allgemeinen eine Reinheit von ≥ 98%, vorzugsweise ≥ 99% und eine APHA-Farbzahl von <70, vorzugsweise <20 APHA. (Siehe Tabelle 1). Als Nebenkomponenten sind hauptsächlich geringe Mengen von Glykol, Vinyloxyethanol und *N*,*N*-Dimethylethanolamin vorhanden, wobei eine geringe Verfärbung, die sich auch nach der Destillation wieder einstellt, im Wesentlichen auf Spuren von Vinyloxyethanol zurückgeht.

In einer bevorzugten Ausführungsform der Erfindung wird daher Vinyloxyethanol von DMAEE abgetrennt oder durch Zugabe von phosphoriger Säure, H₃PO₃, zerstört.

Dabei kann entweder das in Schritt (c) erhaltene DMAEE einer weiteren Destillation unterworfen werden, bei der phosphorige Säure zugesetzt wird, oder die phosphorige Säure wird bereits bei der fraktionierten Destillation zugegeben.
Es ist möglich, die phosphorige Säure kristallin oder als Gemisch von phosphoriger Säure und Wasser einzusetzen. Sowohl kristalline Ware als auch wässrige Lösungen sind kommerziell erhältlich (z.B. bei Supresta oder Honeywell).

Bevorzugt ist die Zugabe eines Gemischs von phosphoriger Säure und Wasser, besonders bevorzugt in einem Gewichts-Verhältnis von 1 : 10 bis 10 : 1, insbesondere von 1 : 1. Im Allgemeinen werden 0,01 bis 10, vorzugsweise 0,25 bis 1 Gewichts-Teile phosphorige Säure pro 25 Gewichts-Teile des zu destillierenden Gemischs eingesetzt.

Der oben beschriebene Vorteil, dass DMAEE auch bei höheren Temperaturen in der Destillation stabil bleibt, wird insbesondere bei dieser bevorzugten Ausführung des Verfahrens genutzt, weil phosphorige Säure H₃PO₃ bei erhöhter Temperatur besonders aktiv für die Zerstörung von Vinyloxyethanol (VOE) ist. Diese Reaktion läuft bei 150°C bis 200°C besonders vorteilhaft ab. Insbesondere bei 180°C bis 190°C sind geringere Mengen an H₃PO₃ erforderlich. Aus diesem Grunde ist eine Destillation bei 400 bis 600 mbar besonders vorteilhaft, weil sich dann bei der Destillation eine Sumpftemperatur von 175 bis 200°C, vorzugsweise 180 bis 190°C, einstellt.

In beiden Varianten wird das Destillationsgemisch bei einer Temperatur von 175 bis 200°C, vorzugsweise 180 bis 190°C und einem Druck von 400 bis 600 mbar, vorzugsweise ungefähr 500 mbar, bei vollständigem Rücklauf getempert, vorzugsweise für 1 h bis 3 h, insbesondere ungefähr 2 h.

Erfolgt die Abtrennung oder Zerstörung des Vinyloxyethanols in einer separaten Destillation, wird diese vorzugsweise über eine Boden-, Füllkörper-, Packungs- oder Trennwandkolonne, besonders bevorzugt Packungs- oder Trennwandkolonne unter den oben angegebenen Druck- und Temperaturbedingungen durchgeführt.

Weitere Nebenkomponenten können nach bekannten, dem Fachmann geläufigen Methoden abgetrennt werden, so kann die Hauptnebenkomponente Glykol beispielsweise durch Zugabe eines geeigneten höheren Aldehyds als schwersiedendes Acetal abgetrennt werden.

Das erhaltene DMAEE eignet sich beispielsweise als Zwischenprodukt zur Synthese von Pharmawirkstoffen oder als Katalysator bei Polyurethan-Herstellung.

Die Erfindung wird durch die Beispiele näher erläutert, ohne sie dadurch einzuschränken.

### Beispiel 1: Destillation des Sumpfstromes einer Kolonne zur Abtrennung von N,N-Dimethylethanolamin

Das Rohprodukt der großtechnischen Umsetzung von Dimethylamin und Ethylenoxid wird zur Abtrennung von *N*,*N*-Dimethylethanolamin einer Destillation unterzogen.

Der Sumpfrückstand der Kolonne hat folgende Zusammensetzung in GC-FI-%:

| | |
|---|---|
| Glykol: | 6 % |
| Vinyloxyethanol: | 4 % |
| *N*,*N*-Dimethylethanolamin: | 16 % |
| Dimethylaminoethoxyethanol: | 74 % |

918 g dieses Rückstands wurden einer Destillation über eine 1 m Füllkörperkolonne (Durchmesser: 60 mm, Füllkörper: 3x3mm) unterzogen. Das Ergebnis ist in Tabelle 1 gezeigt.

**Tabelle 1: Destillation**

| Fraktion | **Fr. 1** | **Fr. 2** | **Fr. 3** | **Fr. 4** | **Fr. 5** | **Fr. 6** | **Fr. 7** | **Fr. 8** |
|---|---|---|---|---|---|---|---|---|
| Farbzahl | 11 APHA | 7 APHA | 26,5 APHA | 25,5 APHA | 7 APHA | 5 APHA | 13 APHA | 68 APHA |
| Gew. in g | 21 | 84 | 66 | 81 | 120 | 128 | 133 | 143 |
| Sumpf-T in °C | 135-142 | 170-178 | 178-182 | 182 | 182-183 | 183 | 174-176 | 196-235 |
| Übergang-T in °C | 66-70 | 98-106 | 158-160 | 160 | 158-159 | 155-156 | 165-167 | 165-112 |
| Druck in mbar | 125-123 | 415-424 | 436-426 | 426-422 | 421 | 420-418 | 308-312 | 312-35 |
| Rück-/Ablauf | 05:02 | 05:02 | 10:01 | 10:02 | 10:02 | 10:02 | 04:02 | 03:02 |
| Zusammensetzung GC-FI-% | | | | | | | | |
| Glykol | 0,00 | 0,00 | 29,53 | 38,85 | 31,92 | 0,37 | 0,00 | 0,00 |
| Vinyloxyethanol | 0,02 | 30,17 | 18,59 | 0,28 | 0,18 | 0,21 | 0,05 | 0,04 |
| Dimethylethanolamin | 99,98 | 69,83 | 6,61 | 0,17 | 0,11 | 0,15 | 0,06 | 0,08 |
| DMAEE | 0,00 | 0,00 | 45,27 | 60,7 | 67,79 | 99,27 | 99, 89 | 99,88 |
| Summe % | 100,00 | 100,00 | 100,00 | 100,00 | 100,00 | 100,00 | 100,00 | 100,00 |
| | | | | | | | | |
| Rückstand | 94 g | | 29,26 | 49,13 | 81,07 | 124,99 | 131,87 | 141,70 |

Das in den Fraktionen 6 bis 8 erhaltene Dimethylaminoethoxyethanol entsprach einer isolierten Ausbeute von etwa 72 %. Die Ausgangsware war sehr dunkel gefärbt. Durch die Destillation konnte die Farbzahl wesentlich verbessert werden.

### Beispiel 2: Destillation über H₃PO₃

Von der Destillation aus Beispiel 1 wurden die Fraktionen 6 und 7 vereinigt und davon 250 g mit einer Mischung aus 2,5 g destilliertem Wasser und 2,5 g phosphoriger Säure H₃PO₃ vermischt. Die homogene Mischung wurde 2 Stunden bei 185°C getempert und anschließend über einer 20 cm Kolonne bei 500 mbar und 182-184 °C destilliert.

**Tabelle 2: Destillation über H₃PO₃**

| | (Fr. 6 + Fr. 7) | Destillat |
|---|---|---|
| Farbzahl (APHA) | 19 | 0 |
| Zusammensetzung GC-FI% | | |
| Glykol | 0,20 | 0,26 |
| Vinyloxyethanol | 0,13 | 0,00 |
| Dimethylethanolamin | 0,12 | 0,08 |
| DMAEE | 99,55 | 99,66 |
| Summe % | 100,00 | 100,00 |

Durch die Behandlung mit phosphoriger Säure und anschließende Destillation wurde nahezu quantitativ farbloses *N*,*N*-Dimethylaminoethoxyethanol (DMAEE) erhalten, welches laut GC kein Vinyloxyethanol mehr enthielt.

### Beispiel 3:

Kombination von Destillation und Entfernung von Vinyloxyethanol in einem einzigen Schritt.

In einer Füllkörperkolonne (Länge: 1 m, Durchmesser: 60 mm, 3x3 mm Füllkörper, Rückflussteiler, ca. 30 theoretische Böden) wurden 250 g einer Rohware mit der folgenden Zusammensetzung in die Blase eingefüllt: 6,85% Glykol, 4,50% Vinyloxyethanol, 15,20% Dimethylethanolamin, 70,70% *N*,*N*-Dimethylaminoethoxyethanol (DMAEE), 0,08% (Dimethylaminoethyl)vinylether.

Die 250 g der Rohware wurden mit einer Mischung aus 10 g destilliertem Wasser und 10 g phosphoriger Säure vermischt. Die Sumpftemperatur in der Kolonne wurde bei vollständigem Rücklauf auf 145°C eingestellt und es wurde 2 h lang zum Rücklauf erhitzt. Nach diesen zwei Stunden wurde dem Kolonnensumpf eine Probe entnommen und folgende Zusammensetzung gefunden:

0,01 % Vinyloxyethanol, 0,1 % Methanol, 1,69% Methyldioxalan, 2,50% Glykol, 6,44% *N*,*N-*Dimethylethanolamin (DMEOA), 1,78% N-Ethyl-N-methylethanolamin und 74,30% *N*,*N-*Dimethylaminoethoxyethanol (DMAEE).

Anschließend wurde bei 500 mbar Kolonnendruck, einem Verhältnis von Rücklauf zu Ablauf von ca. 5:1 - 2:1, 180°C Sumpftemperatur und 30-72°C, dann 72-150°C Übergangstemperatur fraktionierend destilliert. Es wurden ca. 25% Vorlauf abgetrennt, der insbesondere aus Methyldioxalan, *N*-Methylmorpholin, *N*,*N*-Dimethylethanolamin (DMEOA) und Glykol bestand.

Anschließend wurde das Vakuum auf 100 mbar abgesenkt und es wurde bei einer Sumpftemperatur von 138°C ansteigend bis auf 200-250°C, einer Übergangstemperatur von konstant 130°C und einem Verhältnis von Rücklauf zu Ablauf von 4:5 weiterdestilliert. Dabei wurde das Reinprodukt in einer Reinheit von 77-99% erhalten, der Vinyloxyethanol (VOE) - Gehalt betrug in allen Reinfraktionen deutlich weniger als 0,1%. Man erhielt 145 g des Wertprodukts *N*,*N*-Dimethylaminoethoxyethanol (DMAEE), dies entspricht ca. 82% Ausbeute bezogen auf das in der Rohware enthaltene DMAEE (176 g) und 58% Ausbeute bezogen auf die Gesamtmasse der anfangs eingesetzten Rohware (250 g).

## Patentansprüche

1. Verfahren zur Herstellung von *N*,*N*-Dimethylaminoethoxyethanol (DMAEE), wobei man
a) Dimethylamin und Ethylenoxid zur Reaktion bringt,
b) das entstandene Produktgemisch von *N*,*N*-Dimethylethanolamin und DMAEE destillativ auftrennt, wobei eine DMAEE-haltige Fraktion als Sumpfstrom erhalten wird, und
c) DMAEE aus der in (b) erhaltenen Fraktion destillativ abtrennt.

2. Verfahren nach Anspruch 1, wobei die destillative Abtrennung des DMAEE in Schritt b) in einem Temperaturbereich (Sumpf) von 40 bis 150 °C und bei einem Druck von 5 bis 1050 bar durchgeführt wird.

3. Verfahren nach Anspruch 1 oder 2, wobei die fraktionierte Destillation in Schritt c) über eine Kolonne erfolgt.

4. Verfahren nach Anspruch 3, wobei die fraktionierte Destillation in einem Temperaturbereich (Sumpf) von 40 bis 280 °C und einem Druck von 1 bis 1000 mbar erfolgt.

5. Verfahren nach Anspruch 1 oder 2, wobei die fraktionierte Destillation in Schritt c) über einen Dünnschichtverdampfer, Fallfilmverdampfer oder Kurzwegverdampfer, gegebenenfalls mit aufgesetzter Kolonne erfolgt.

6. Verfahren nach Anspruch 5, wobei die Destillation bei einer Übergangstemperatur von 40 bis 150 °C und bei einem Druck von 0,001 bis 1 mbar erfolgt.

7. Verfahren nach einem oder mehreren der Ansprüche 1 bis 6, wobei dem Destillationsgemisch in Schritt (c) phosphorige Säure zugesetzt wird oder das in Schritt (c) erhaltene DMAEE in einem weiteren Schritt über phosphoriger Säure destilliert wird.

8. Verwendung des Sumpfstromes einer Destillation der Reaktionsmischung von Dimethylamin und Ethylenoxid zur Gewinnung von DMAEE.

9. Vorrichtung zur Durchführung des Verfahrens nach einem oder mehreren der Ansprüche 1 bis 7, enthaltend (I) einen Rohreaktor mit Zuleitungen für Dimethylamin und Ethylenoxid sowie einer Ableitung für das Reaktionsprodukt, (II) eine Destillationsvorrichtung zur Anreicherung des DMAEE durch destillative Abtrennung von *N*,*N-*Dimethylethanolamin aus dem Reaktionsgemisch mit einer Ableitung des DMAEEhaltigen Sumpfproduktes am Fuß der Destillationsvorrichtung und (III) eine zur fraktionierten Destillation geeignete Vorrichtung mit einer Zuleitung für das DMAEE-haltige Reaktionsgemisch am Fuß oder an der Seite und einer Ableitung für das destillierte DMAEE im Kopfbereich der Vorrichtung.

10. Vorrichtung nach Anspruch 9, wobei die zur fraktionierten Destillation geeignete Vorrichtung (III) eine Kolonne oder ein Dünnschicht-, Fallfilm- oder Kurzwegverdampfer, die drei letztgenannten jeweils gegebenenfalls mit aufgesetzter Kolonne, ist.

## Claims

1. A process for preparing *N*,*N-*dimethylaminoethoxyethanol (DMAEE), wherein
a) dimethylamine and ethylene oxide are reacted,
b) the resulting product mixture of *N*,*N-*dimethylethanolamine and DMAEE is separated by distillation to obtain a DMAEE-containing fraction as the bottom stream, and
c) DMAEE from the fraction obtained in (b) is removed by distillation.

2. The process according to claim 1, wherein the distillative removal of the DMAEE in step b) is performed within a temperature range (bottom) of from 40 to 150°C and at a pressure of from 5 to 1050 bar.

3. The process according to claim 1 or 2, wherein the fractional distillation in step c) is effected by means of a column.

4. The process according to claim 3, wherein the fractional distillation is effected within a temperature range (bottom) of from 40 to 280°C and a pressure of from 1 to 1000 mbar.

5. The process according to claim 1 or 2, wherein the fractional distillation in step c) is effected by means of a thin-film evaporator, falling-film evaporator or short-path evaporator, optionally with an attached column.

6. The process according to claim 5, wherein the distillation is effected at a distillation temperature of from 40 to 150°C and at a pressure of from 0,001 to 1 mbar.

7. The process according to one or more of claims 1 to 6, wherein phosphorous acid is added to the distillation mixture in step (c) or the DMAEE obtained in step (c) is distilled over phosphorous acid in a further step.

8. The use of the bottom stream of a distillation of the reaction mixture of dimethylamine and ethylene oxide for obtaining DMAEE.

9. An apparatus for performing the process according to one or more of claims 1 to 7, comprising (I) a tubular reactor with inlets for dimethylamine and ethylene oxide and an outlet for the reaction product, (II) a distillation apparatus for enriching the DMAEE by distillatively removing *N,N-*dimethylethanolamine from the reaction mixture with an outlet for the DMAEE-containing bottom product at the bottom of the distillation apparatus and (III) an apparatus which is suitable for fractional distillation and has an inlet for the DMAEE-containing reaction mixture at the bottom or at the side and an outlet for the distilled DMAEE in the top region of the apparatus.

10. The apparatus according to claim 9, wherein the apparatus (III) suitable for fractional distillation is a column or a thin-film evaporator, falling-film evaporator or short-path evaporator, the last three in each case optionally with an attached column.

## Revendications

1. Procédé de fabrication de *N*,*N-*diméthylaminoéthoxyéthanol (DMAEE), dans lequel
a) de la diméthylamine et de l'oxyde d'éthylène sont mis en réaction,
b) le mélange de produits formé de *N*,*N-*diméthyléthanolamine et de DMAEE est séparé par distillation, une fraction contenant du DMAEE étant obtenue en tant que courant de fond, et
c) le DMAEE est séparé par distillation de la fraction obtenue en (b).

2. Procédé selon la revendication 1, dans lequel la séparation distillative du DMAEE à l'étape b) est réalisée dans une plage de températures (de fond) de 40 à 150 °C et à une pression de 5 à 1 050 bar.

3. Procédé selon la revendication 1 ou 2, dans lequel la distillation fractionnée à l'étape c) a lieu dans une colonne.

4. Procédé selon la revendication 3, dans lequel la distillation fractionnée a lieu dans une plage de températures (de fond) de 40 à 280 °C et à une pression de 1 à 1 000 mbar.

5. Procédé selon la revendication 1 ou 2, dans lequel la distillation fractionnée à l'étape c) a lieu dans un évaporateur à couche mince, un évaporateur à film tombant ou un évaporateur à court trajet, éventuellement avec une colonne superposée.

6. Procédé selon la revendication 5, dans lequel la distillation a lieu à une température de transition de 40 à 150 °C et à une pression de 0,001 à 1 mbar.

7. Procédé selon une ou plusieurs des revendications 1 à 6, dans lequel de l'acide phosphoreux est ajouté au mélange de distillation à l'étape (c) ou le DMAEE obtenu à l'étape (c) est distillé lors d'une étape supplémentaire par de l'acide phosphoreux.

8. Utilisation du courant de fond d'une distillation du mélange réactionnel de diméthylamine et d'oxyde d'éthylène pour l'obtention de DMAEE.

9. Dispositif pour la réalisation du procédé selon une ou plusieurs des revendications 1 à 7, contenant (I) un réacteur tubulaire muni de conduites d'alimentation pour la diméthylamine et l'oxyde d'éthylène et d'une conduite de déchargement pour le produit de réaction, (II) un dispositif de distillation pour la concentration du DMAEE par séparation distillative de *N*,*N*-diméthyléthanolamine du mélange réactionnel, muni d'une conduite de déchargement du produit de fond contenant du DMAEE en bas du dispositif de distillation, et (III) un dispositif approprié pour la distillation fractionnée, muni d'une conduite d'alimentation pour le mélange réactionnel contenant du DMAEE en bas ou sur le côté et d'une conduite de déchargement pour le DMAEE distillé dans la zone de tête du dispositif.

10. Dispositif selon la revendication 9, dans lequel le dispositif (III) approprié pour la distillation fractionnée est une colonne ou un évaporateur à couche mince, à film tombant ou à court trajet, les trois derniers chacun éventuellement avec une colonne superposée.
